Europäisches Patentamt

European Patent Office

Office européen des brevets

(19)

(11) Publication number: **0 045 627**
**B1**

(12) **EUROPEAN PATENT SPECIFICATION**

(45) Date of publication of patent specification: **27.02.85**

(51) Int. Cl.⁴: **F 16 L 13/00,** F 16 B 4/00,
B 23 P 11/02

(21) Application number: **81303486.5**

(22) Date of filing: **30.07.81**

(54) Enhanced recovery memory metal device.

| | |
|---|---|
| (30) Priority: **31.07.80 US 173948** | (73) Proprietor: **RAYCHEM CORPORATION**<br>**300 Constitution Drive**<br>**Menlo Park California 94025 (US)** |
| (43) Date of publication of application:<br>**10.02.82 Bulletin 82/06** | |
| | (72) Inventor: **Broyles, Harry Clifton**<br>**260 North Pastoria Avenue**<br>**Sunnyvale California 94086 (US)** |
| (45) Publication of the grant of the patent:<br>**27.02.85 Bulletin 85/09** | |
| (84) Designated Contracting States:<br>**AT BE CH DE FR IT LI NL SE** | (74) Representative: **Jones, David Colin et al**<br>**Raychem Limited Intellectual Property Law**<br>**Department Swan House 37-39 High Holborn**<br>**London WC1 (GB)** |
| (56) References cited:<br>**EP-A-0 017 677**<br>**EP-A-0 030 119**<br>**DE-A-2 448 160**<br>**DE-A-2 900 518**<br>**GB-A-1 554 431**<br>**GB-A-1 554 432**<br>**GB-A-1 554 433**<br>**US-A-4 035 007**<br>**US-A-4 099 991**<br>**US-A-4 197 880**<br><br>**SCIENTIFIC AMERICAN, Vol. 241, Nr. 5,**<br>**November 1979, New York L.M. SCHETKY**<br>**"Shape-Memory Alloys"** | |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a recoverable device, and in particular to a recoverable device comprising a memory metal.

Memory metals are alloys which manifest the shape — memory effect — such alloys are well known and they, and the shape — memory effect, are discussed in, for example, "Shape — Memory Alloys", Scientific American, v. 281, pp 74/82 (Nov. 1979).

In the past memory metals have been used in mechanical composite pipe couplings to force tensile load bearing members into gripping contact with pipes and tubing. It is known to use memory metals as drivers, in this way, both externally and internally with respect to the pipes. The recovery capability of the memory metal in such cases is, however, limited to the inherent recovery of the memory metal comprising the drivers.

GB—A—1554432 discloses a coupling device comprising a memory metal member and a deformable member in which the memory metal member is preferably provided with a bending deformation, optionally in addition to a compressive or tensioning deformation.

It is to be understood that, as used herein, the term "inherent recovery" refers to the extent of recovery available when a memory metal article, which has been deformed in simple tension or compression while in a martensitic state, returns to the austenitic state in the absence of restraining forces. Although this inherent recovery is theoretically high for single crystals, in practice the inherent recovery of a polycrystalline memory metal is between about 4% and 9% maximum. Thus for the solid memory metal drivers, used hitherto in pipe couplings, recovery is limited to between about 4% and 9%.

It is an object of the present invention to provide a memory metal device having recoverability that is enhanced beyond the inherent recovery of the memory metal.

In accordance with the present invention there is provided a device comprising a recoverable sheet of memory metal having a plurality of perforations therein, the shape and/or pattern of the perforations being arranged to enhance the recovery of the sheet in at least one direction within the plane of the sheet beyond the inherent recovery of the memory metal.

A sheet, that is to say an essentially two-dimension configuration, is understood to define a plane, and reference made herein to the plane of a sheet includes the surface of the sheet even when it is arranged in a non-planar, for example cylindrical or conical, configuration.

In one embodiment of the invention the perforations are arranged in a rhombic lattice pattern, the direction of enhanced recovery being along one of the diagonals of the rhombic lattice.

According to another embodiment of the invention, the perforations are generally rectilinear in shape, preferably rectangular or square, with the width of the rectangle extending along the direction of enhanced recovery.

Two embodiments of a device, each in accordance with the present invention will now be described, by way of example, with reference to the accompanying drawings, in which:—

Figure 1 is a plan view showing a first embodiment of the device, before recovery;

Figure 2 is a plan view showing the embodiment of Figure 1 after recovery;

Figure 3 is a plan view showing an alternative embodiment of the device before recovery; and

Figure 4 is a plan view showing the device of Figure 3 after recovery.

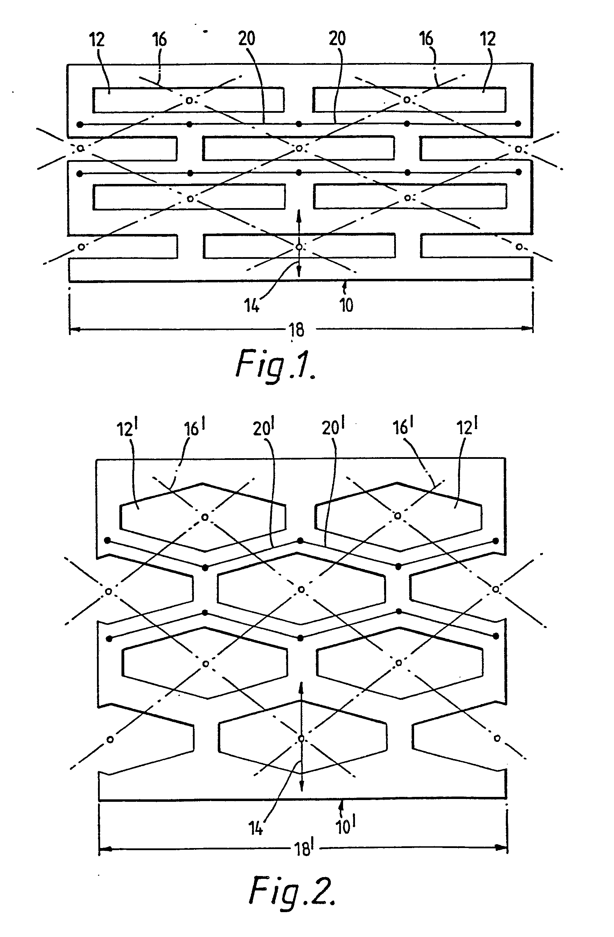

Referring to the drawings, Figure 1 illustrates a device comprising a sheet 10 having a plurality of perforations 12 contained therein. The sheet 10 is made from a memory metal, that is an alloy which manifests the shape — memory effect as discussed hereinbefore. Especially suitable as alloys for sheet 10 are ternary or quaternary alloys of copper, zinc, aluminium and/or manganese, and also nickel-titanium binary or ternary alloys. The plurality of perforations 12 are of a particular shape and are arranged in a particular pattern, so as to enhance the recovery of the sheet 10 in substantially a single direction 14, within the plane of sheet 10. Figure 2 illustrates the sheet 10 after it has recovered in direction 14.

The geometric arrangement of the sheet 10 and the perforations 12 shown in Figure 1 and the corresponding arrangement of the sheet 10' and the perforations 12' in Figure 2 represent the device respectively before and after recovery has been effected by heating, before heating, in a compressed or non-expanded condition, and after heating in a recovered or expanded condition. The sheet 10 of Figure 1 therefore represents a compressed member which expands on heating.

It should be appreciated that the effect of heating can be reversed. Such a situation may be envisaged by considering Figure 2 as representing a sheet 10' having perforations 12' before heating, the sheet 10' and the perforations 12' being in an expanded condition which upon heating recover to the geometric arrangement shown in Figure 1 wherein sheet 10 is non-expanded.

The perforations 12 in sheet 10 of Figure 1 are generally rectangular in shape and are arranged in a rhombic lattice pattern. The rhombic lattice is clearly shown by the centre lines 16 which interconnect the geometric centres of the perforations 12. An expansion or contraction in the direction 14 constitutes an expansion or contraction in a direction along a diagonal of the rhombic lattice. In Figure 2, centre lines 16' interconnecting the perforations 12' likewise define a rhombic lattice, the direction of recovery 14' again being along a diagonal of the lattice.

The width of the generally rectangular perforations 12 is relatively narrow and extends along the direction 14. This construction enables the perforations 12 to recover to the substantially flat diamond shaped perforations 12' in Figure 2. The geometric interrelationship between the perforations 12 in Figure 1 and the perforations 12′ in Figure 2 represents a considerable recovery in the direction 14. The initial length 18 of sheet 10 in Figure 1 is decreased to a shorter length 18′ in Figure 2 upon recovery of the sheet 10, and it will be appreciated that there is a geometric relationship between the width/length ratio of the perforations 12 and the change in length 18 to 18′ of the sheet 10. A change in the ratio of width/length of the perforations 12 changes the amount of recovery and therefore the difference in length between 18 and 18′. Thus this width/length ratio may be varied to create sheets which are capable of enhanced motion in more than one direction within the plane of said sheet, for example, expansion in one direction and contraction in the perpendicular direction.

The device of Figures 1 and 2 can likewise be viewed as a two dimensional array of co-acting lever arms within the plane of sheet 10. These co-acting arms are represented in Figure 1 as centre line segments 20 of sheet 10. It can be seen that the co-acting arms 20 of Figure 1 are bent into the co-acting arms 20′ of Figure 2 on recovery.

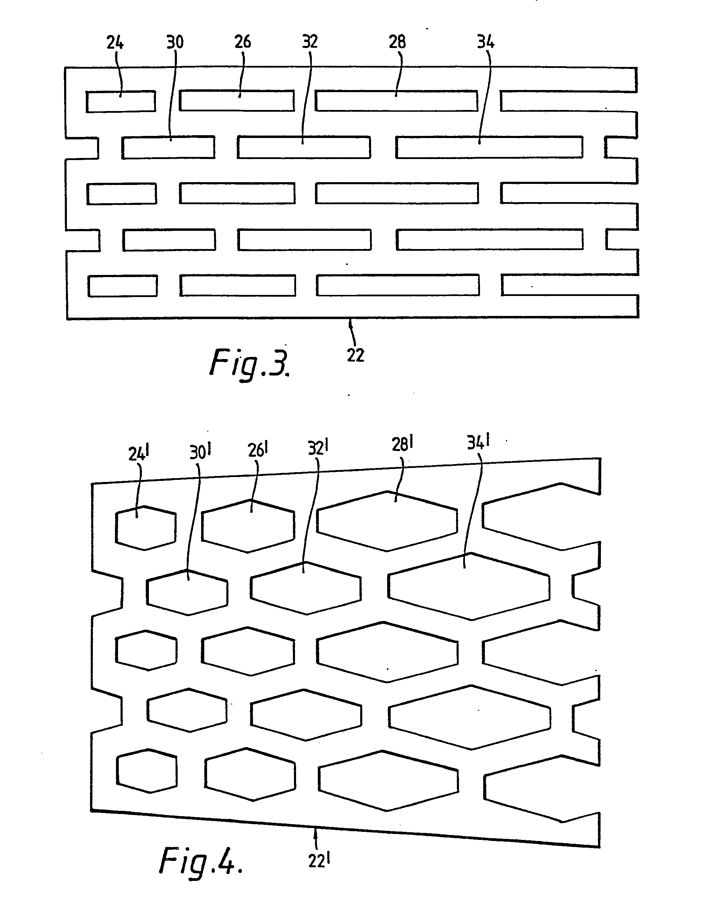

Figure 3 illustrates a sheet 22 having variously sized perforations 24, 26 and 28; perforation 26 being longer than perforation 24 and perforation 28 being longer than perforation 26, the perforations being arranged generally in a line in order of increasing length. A similar increasing length relationship exists with regard to the perforations 30, 32 and 34. The perforations 24 to 34 are arranged in a particular pattern.

Figure 4 illustrates the device of Figure 3 after recovery where the variously sized perforations 24 to 34 have recovered to similar diamond shaped perforations 24′ to 34′. It will be seen by comparing Figures 3 and 4 that the size of the diamond shaped perforations after recovery depends on the length of the corresponding rectangular perforations before recovery; the longer the rectangular perforation the larger the diamond shaped perforation. Thus the extent of the recovery of the sheet, which is substantially in a single direction, is different at different positions along the sheet in the direction perpendicular to its direction or recovery. In the embodiment shown in Figures 3 and 4, this variable extent of recovery is such that the sheet 22 recovers from a generally rectangular configuration, as shown in Figure 3, to a generally fan shaped configuration, as shown in Figure 4, but it will be appreciated that other arrangements of perforations would produce a different overall shape change of the sheet.

Figures 1 to 4 illustrate structures which enhance the available recovery motion of a memory metal. The particular pattern illustrated in Figure 2 may be compressed or reduced by as much as 25% to 75% in the direction 14, or stated another way, the pattern illustrated in Figure 1 allows for a memory metal structure to expand some 50 to 300% in the direction 14.

In general the amount of recoverable motion available is a function of the perforations slot width, length spacing and the amount of bending strain per lever arm. These variables can be balanced in various combinations to yield similar or identical performance. Likewise, the shape of the perforations will vary in compressed or non-expanded condition from substantially rectangular to oval. The perforations may, before recovery be substantially rectilinear, preferably rectangular or square. The corners of a rectangular shape or radius help to accommodate bending stress concentrations. The perforations may be machined, etched, stamped, or otherwise formed.

For practical applications the sheet illustrated in Figures 1 and 2 may be used in a planar configuration or may be formed into a generally cylindrical configuration. A use for such a configuration is disclosed in our contemporaneously filed application, publication number EP—A—0045629.

The sheets illustrated in Figures 1 to 4 may also be formed into generally conical configurations or, indeed into almost any three-dimensional geometric configuration to take advantage of the one- or two-dimensional recovery of the sheets within their planes. The direction of recovery of the device may be substantially radially or axially of the cone, or as the case may be, of the cylinder.

The sheets illustrated in Figures 1 to 4, and the various configurations that can be made from said sheets may also be used as drivers in known pipe coupling and sealing arrangements such as are described in, for example, British Patent Number 1554431 and US Patent Number 4197880.

It is an advantage of the present invention that a single sheet may be formed and used in such a variety of configurations and hence for a variety of purposes.

It is a further advantage of the device of the present invention that it can be formed into a continuous configuration, for example, a cylinder, and can thus impart a uniform pressure around its surface.

## Claims

1. A device comprising a recoverable sheet (10) of memory metal having a plurality of perforations (12) therein, the shape and/or pattern of the perforations being arranged so as to enhance the recovery of the sheet in at least one direction within the plane of the sheet beyond the inherent recovery of the memory metal.

2. A device according to Claim 1, wherein the sheet is recoverable substantially only in a single direction (14).

3. A device according to Claim 1 or 2, wherein the perforations are arranged in a rhombic lattice pattern and the direction (14) of recovery of said sheet is substantially along a diagonal of said lattice.

4. A device according to any preceding claim, wherein a two-dimensional array of co-acting lever arms within the plane of the sheet is defined by said perforations, and wherein the recovery of the sheet comprises bending of said lever arms.

5. A device according to any preceding claim, wherein said sheet is substantially planar.

6. A device according to any one of Claims 1 to 4, wherein said sheet is arranged in a substantially cylindrical configuration.

7. A device according to any one of Claims 1 to 4, wherein said sheet is arranged in a substantially conical configuration.

8. A device according to Claim 6 or 7, wherein the direction of recovery of said device is substantially radially or axially of the cylinder or cone.

9. A device according to any preceding claim, wherein the extent of recovery of the sheet is different at different positions along the sheet in the direction perpendicular to the direction of recovery.

10. A device according to any preceding claim, wherein the shape of said perforations is substantially rectilinear, preferably rectangular or square.

11. A device according to any preceding claim, wherein said enhanced recovery of the sheet is effected in two directions within the sheet.

## Revendications

1. Un dispositif comprenant une feuille (10) à reprise de forme en métal à mémoire dans laquelle sont formées une série de perforations (12), la forme et/ou la disposition des perforations étant agencées pour accroître la reprise de la feuille dans au moins une direction à l'intérieur du plan de la feuille au-delà de la reprise inhérente du métal à mémoire.

2. Un dispositif selon la revendication 1, dans lequel la reprise de la feuille ne peut s'effectuer que dans une unique direction (14).

3. Un dispositif selon la revendication 1 ou 2, dans lequel les perforations sont agencées suivant une configuration de réseau rhombique et la direction (14) de reprise de ladite feuille est sensiblement suivant une diagonale dudit réseau.

4. Un dispositif selon une quelconque revendication précédente, dans lequel un ensemble bidimensionnel de bras de levier coopérants à l'intérieur du plan de la feuille est défini par lesdites perforations et dans lequel la reprise de la feuille entraîne la flexion desdits bras de levier.

5. Un dispositif selon une quelconque revendication précédente, dans lequel ladite feuille est sensiblement plane.

6. Un dispositif selon l'une quelconque des revendications 1 à 4, dans lequel ladite feuille est mise dans une configuration sensiblement cylindrique.

7. Un dispositif selon l'une quelconque des revendications 1 à 4, dans lequel ladite feuille est mise dans une configuration sensiblement conique.

8. Un dispositif selon la revendication 6 ou 7, dans lequel la direction de reprise dudit dispositif est la direction sensiblement radiale ou axiale du cylindre ou du cône.

9. Un dispositif selon une quelconque revendication précédente, dans lequel l'importance de la reprise de la feuille est différente à différentes positions de la feuille dans la direction perpendiculaire à la direction de reprise.

10. Un dispositif selon une quelconque revendication précédente, dans lequel la forme desdites perforations est sensiblement rectiligne, de préférence rectangulaire ou carrée.

11. Un dispositif selon une quelconque revendication précédente, dans lequel ladite reprise accrue de la feuille est effectuée dans deux directions à l'intérieur de la feuille.

## Patentansprüche

1. Vorrichtung mit einem rückstellfähigen flächenhaften Material (10) aus Memory-Metall, das in sich eine Anzahl von Perforationen (12) aufweist, wobei die Form und/oder das Muster der Perforationen ausgebildet, sind derart, dass die Rückstellung des flächenhaften Materials in mindestens einer Richtung innerhalb der Ebene des flächenhaften Materials über die inhärente Rückstellung des Memory-Metalls hinaus erhöht ist.

2. Vorrichtung nach Anspruch 1, bei der das flächenhafte Material im wesentlichen nur in einer einzigen Richtung (14) rückstellbar ist.

3. Vorrichtung nach Anspruch 1 oder 2, bei der die Perforationen in einem rhombischen Gittermuster angeordnet sind und die Richtung (14) der Rückstellung des flächenhaften Materials im wesentlichen längs einer Diagonale des Gitters verläuft.

4. Vorrichtung nach einem vorhergehenden Anspruch, bei der die Perforationen eine zweidimensionale Gruppierung zusammenwirkender Hebelarme in der Ebene des flächenhaften Materials definieren und bei der die Rückstellung des flächenhaften Materials eine Biegung jener Hebelarme umfasst.

5. Vorrichtung nach einem vorhergehenden Anspruch, bei der das flächenhafte Material im wesentlichen eben ist.

6. Vorrichtung nach einem der Ansprüche 1 bis 4, bei der das flächenhafte Material zu einer im wesentlichen zylinderförmigen Konfiguration ausgebildet ist.

7. Vorrichtung nach einem der Ansprüche 1 bis 4, bei der das flächenhafte Material zu einer im wesentlichen konischen Konfiguration ausgebildet ist.

8. Vorrichtung nach Anspruch 6 oder 7, bei der die Richtung ihrer Rückstellung im wesentlichen radial oder axial zu dem Zylinder oder Kegel verläuft.

9. Vorrichtung nach einem vorhergehenden Anspruch, bei der das Ausmass der Rückstellung des flächenhaften Materials an verschiedenen Stellen längs des flächenhaften Materials in der Richtung senkrecht zur Rückstellrichtung verschieden ist.

10. Vorrichtung nach einem vorhergehenden Anspruch, bei der die Form der Perforationen im wesentlichen geradlinig, vorzugsweise rechteckig oder quadratisch ist.

11. Vorrichtung nach einem vorhergehenden Anspruch, bei der die erhöhte Rückstellung des flächenhaften Materials in zwei Richtungen innerhalb des flächenhaften Materials ausgebildet ist.

**0 045 627**

Fig.1.

Fig.2.

Fig.3.

Fig.4.